(19) Europäisches Patentamt
European Patent Office
Office européen des brevets

(11) Veröffentlichungsnummer: **0 024 611**
**B1**

# (12) EUROPÄISCHE PATENTSCHRIFT

(45) Veröffentlichungstag der Patentschrift: **12.01.83**

(51) Int. Cl.³: **C 07 C 45/55,** C 07 C 47/55, C 07 C 47/575, C 07 F 9/40

(21) Anmeldenummer: **80104714.3**

(22) Anmeldetag: **09.08.80**

(54) **Verfahren zur Herstellung von substituierten Benzaldehyden.**

(30) Priorität: **22.08.79 DE 2934034**

(43) Veröffentlichungstag der Anmeldung: **11.03.81 Patentblatt 81/10**

(45) Bekanntmachung des Hinweises auf die Patenterteilung: **12.01.83 Patentblatt 83/2**

(84) Benannte Vertragsstaaten: **BE CH DE FR GB IT LI NL**

(56) Entgegenhaltungen:
**DE-A-2 709 264**

**CHEMISCHE BERICHTE, Band 103, Seiten 2984–2986, Nr. 9, 1970 L. HORNER et al.: «Notiz über die reduktive Umwandlung von Carbonsäuren in ihre Aldehyde»**

**CHEMICAL ABSTRACTS, Band 69, Nr. 17, 21. Oktober 1968, Seite 6307, Nr. 67465y Columbus, Ohio, U.S.A. V. ABRAMOV et al.: "Reactions of dialkyl phosphonates with aldehydes and ketones. XXVIII. Esters of alphahydroxy-beta-chloroethylphosphonic acids and alpha, beta-epoxyethylphosphonic acids"**

(73) Patentinhaber: **BAYER AG, Zentralbereich Patente, Marken und Lizenzen, D-5090 Leverkusen 1, Bayerwerk (DE)**

(72) Erfinder: **Hoffmann, Hellmut, Prof. Dr., Tersteegenweg 17, D-5600 Wuppertal 1 (DE)**

(56) Entgegenhaltungen:
**COMPTES RENDUS, Hebdomadaires, Des séances de l'académie des sciences, Tome deux cent soixanteseizième, Série C: Sciences Chimiques, troisième partie: mai-juin 1973 G. STURTZ et al.: «Chimie Organique. Action du n-butyllithium sur quelques dialkyl et bis-diméthylamido phosphates de benzyle substitué. Réarrangement phosphate-phosphonate»**

**EP 0 024 611 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von substituierten Benzaldehyden

Die Erfindung betrifft ein neues Verfahren zur Herstellung von substituierten Benzaldehyden.

Es ist bekannt, dass man bestimmte substituierte Benzaldehyde, wie z.B. 4-Fluor-3-phenoxy-benzaldehyd, erhält, wenn man Benzylhalogenide, wie z.B. 4-Fluor-3-phenoxy-benzylbromid, mit Hexamethylentetramin umsetzt und das Produkt dieser Umsetzung mit Säuren erhitzt (vergleiche DE-OS 2 709 264).

Bei dieser Synthesemethode erhält man jedoch eine Reihe von substituierten Benzaldehyden nur in geringen Ausbeuten; unbefriedigend sind die Ausbeuten auch bei der Synthese der als Ausgangsstoffe zu verwendenden Benzyl-halogenide, beispielsweise durch Umsetzung von Phenoxy-toluolen mit N-Brom-succinimid.

In Comptes Rendus Hebdomadaires, Band 276, Serie C - 1807ff. wird die basische Hydrolyse eines in α-Stellung alkylierten Oxanions eines Phosphoramids beschrieben. In einem Reaktionsschema wird dabei die intermediäre Bildung von Benzaldehyd postuliert. Dieser Literaturstelle kann jedoch kein Hinweis darauf entnommen werden, ob und unter welchen Bedingungen aus α-Hydroxyphosphonestern Aldehyde gebildet werden.

In Chem. Berichte, Band 103, S. 2984–2986 wird erwähnt, dass α-Ketophosphonsäureester mit Hilfe von Natriumborhydrid zu den α-Hydroxyphosphonestern reduziert werden und daraus in alkalischem Medium die entsprechenden Aldehyde freigesetzt werden können. Entscheidend für eine gute Aldehydausbeute scheint jedoch zu sein, dass die α-Hydroxyphosphonsäureester aus den α-Ketophosphonsäureestern in Gegenwart eines hohen Überschusses an Natriumborhydrid hergestellt werden. Wird mit einer geringeren Menge Natriumborhydrid gearbeitet, sinkt die Ausbeute.

Die vorliegende Erfindung betrifft:

1. ein Verfahren zur Herstellung von substituierten Benzaldehyden der Formel (I)

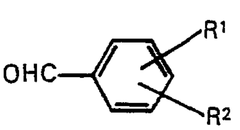 (I)

in welcher
$R^1$ für Wasserstoff oder Halogen steht und
$R^2$ für Halogen oder gegebenenfalls halogensubstituiertes Phenoxy steht, indem man α-Hydroxy-benzyl-phosphonsäurester der Formel II

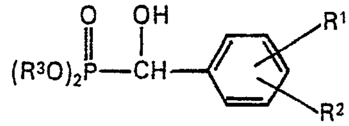 (II)

in welcher
$R^1$ und $R^2$ die oben angegebenen Bedeutungen haben und die beiden Reste
$R^3$ einzeln für Alkyl oder Phenyl oder zusammen für Alkandiyl (Alkylen) stehen,
mit Alkali- oder Erdalkalihydroxiden in einem Zweiphasenmedium aus Wasser und einem mit Wasser nicht mischbaren Lösungsmittel bei Temperaturen zwischen 0 und 100°C umsetzt.

α-Hydroxy-benzyl-phosphonsäureester der Formel (II) (oben) werden hergestellt, indem man Benzoylphosphonsäureester der Formel III

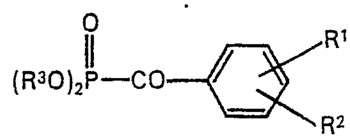 (III)

in welcher
$R^1$, $R^2$ und $R^3$ die unter (1) angegebenen Bedeutungen haben,
mit Hydridkomplexen der Formel

$$M(M'H_4) \qquad (IV)$$

in welcher
M für Lithium, Natrium oder Kalium steht und
M′ für Bor oder Aluminium steht,
gegebenenfalls in Gegenwart eines Puffermittels und gegebenenfalls unter Verwendung von Verdünnungsmitteln bei Temperaturen zwischen −20 und +50°C umsetzt.

Überaschenderweise können nach dem erfindungsgemässen Verfahren 1), verbunden mit dem Verfahren zur Herstellung der Ausgangsverbindungen, die substituierten Benzaldehyde (I) wesentlich einfacher, kostengünstiger und in besseren Ausbeuten als nach bekannten Verfahren hergestellt werden.

Verwendet man als Ausgangsstoffe zur Herstellung der α-Hydroxyphosphonsäureester («Verfahren (3)») beispielsweise 3-Phenoxy-benzoyl-phosphonsäuredimethylester und Natriumtetrahydridoborat und bei der Umsetzung des hierbei gebildeten α-Hydroxy-3-phenoxybenzyl-phosphonsäure-dimethylester nach dem unter 1) dargelegten Verfahren («Verfahren 1)») Kaliumhydroxid als Reaktionskomponente, so kann der Reaktionsablauf durch folgendes Formelschema skizziert werden:

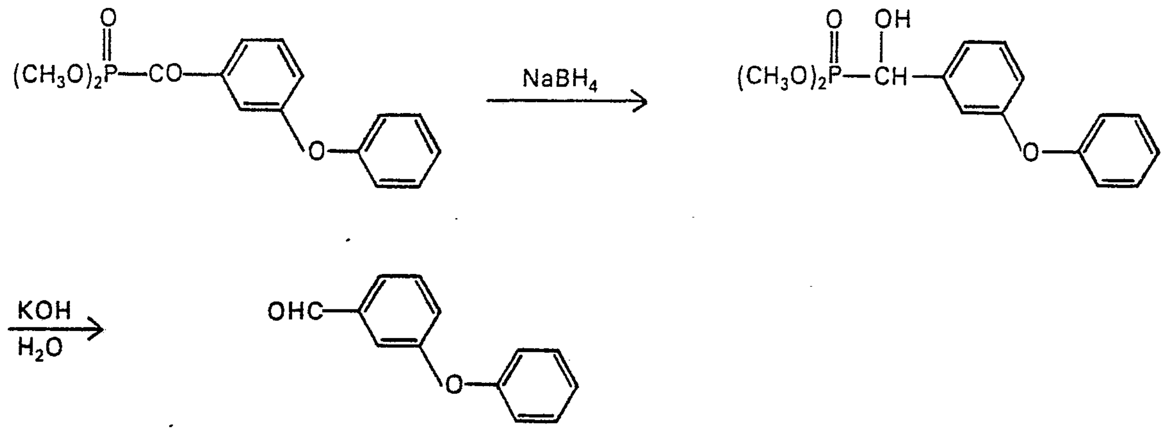


Verfahren 1) wird in einem Zweiphasensystem aus Wasser und mit Wasser nicht mischbaren Lösungsmitteln, wie z.B. Hexan, Heptan, Cyclohexan, Methylenchlorid, Toluol oder Xylol durchgeführt. Alkali- bzw. Erdalkalihydroxide, welche bei Verfahren 1) eingesetzt werden können, sind z.B. Natrium-, Kalium-, Magnesium- und Calciumhydroxid. Vorzugsweise wird Natriumhydroxid verwendet.

Die Temperatur wird bei Verfahren 1) zwischen 0 und 100°C, vorzugsweise zwischen 10 und 50°C gehalten. Das Verfahren wird im allgemeinen bei Normaldruck durchgeführt.

Zur Durchführung von Verfahren 1) werden die Verbindungen der Formel (II) und die Alkali- bzw. Erdalkalihydroxide in etwa äquimolaren Mengen eingesetzt. In einer bevorzugten Ausführungsform wird eine wässrige Alkali- oder Erdalkalihydroxidlösung unter einer Inertgasatmosphäre, wie z.B. unter Stickstoff, vorgelegt, ein mit Wasser nicht mischbares Lösungsmittel und eine Verbindung der Formel (II) dazugegeben und das Gemisch mehrere Stunden gerührt. Zur Aufarbeitung, welche nach üblichen Methoden durchgeführt werden kann, wird beispielsweise mit einem mit Wasser nicht mischbaren Lösungsmittel, wie z.B. Toluol, extrahiert; der Extrakt wird getrocknet, filtriert und eingeengt. Das zurückbleibende Rohprodukt kann durch Vakuumdestillation gereinigt werden.

Die bei Verfahren 1) als Ausgangsverbindungen zu verwendenden α-Hydroxy-benzyl-phosphonsäureester sind durch Formel (II) definiert. Vorzugsweise stehen darin $R^1$ für Wasserstoff oder Fluor in p-Stellung und $R^2$ für Brom oder gegebenenfalls fluor-substituiertes Phenoxy in m-Stellung und $R^3$ einzeln für $C_1$–$C_4$-Alkyl oder Phenyl oder zusammen für 2,2-Dimethyl-propan-1,3-diyl.

Als Beispiele für die Zwischenprodukte der Formel II seien genannt:

α-Hydroxy-3-phenoxy-benzyl,
α-Hydroxy-3-brom-4-fluorbenzyl,
α-Hydroxy-4-fluor-3-phenoxy-benzyl-,
α-Hydroxy-3-(4-fluor-phenoxy)-benzyl- und
α-Hydroxy-4-fluor-3-(4-fluor-phenoxy)-benzyl-phosphonsäuredimethylester und -diethylester sowie
2-Oxo-2-(α-hydroxy-3-phenoxy-benzyl)-,
2-Oxo-2-(α-hydroxy-4-fluor-3-phenoxy-benzyl)-,
2-Oxo-2-(α-hydroxy-3-(4-fluor-phenoxy)-benzyl)- und
2-Oxo-2-(α-hydroxy-4-fluor-3-(4-fluor-phenoxy)-benzyl-5,5-dimethyl-1,3,2-dioxaphosphorinan.

Die Verbindungen der Formel II sind zum Teil neu.

Verfahren 3) zur Herstellung der Verbindungen der Formel (II) wird vorzugsweise unter Verwendung von Verdünnungsmitteln durchgeführt. Als solche kommen beispielsweise Wasser und/oder Alkohole, wie z.B. Methanol oder Ethanol in Frage. Besonders bevorzugt werden Zweiphasensysteme aus Wasser und mit Wasser nicht mischbaren Lösungsmitteln, wie z.B. Hexan, Heptan, Cyclohexan, Methylenchlorid, Chloroform, Ethylenchlorid, Diethylether, Toluol oder Xylol verwendet.

Puffermittel, welche beim Verfahren 3) zum Halten des pH-Wertes bei ca. 6–7 verwendet werden können, sind z.B. Natriumdihydrogenphosphat und Kaliumdihydrogenphosphat.

Als Beispiele für die Hydridkomplexe der Formel (IV), welche bei Verfahren 3) als Reduktionsmittel zu verwenden sind, seien Lithiumtetrahydridoaluminat (Lithiumalanat), sowie Natriumtetrahydridoborat (Natriumboranat) genannt. Letzteres wird vorzugsweise verwendet.

Die Reaktionstemperatur wird bei Verfahren 3) zwischen −20 und +50°C, vorzugsweise zwischen −10 und +30°C gehalten. Das Verfahren wird im allgemeinen bei Normaldruck durchgeführt.

Zur Durchführung von Verfahren 3) werden auf 1 Mol Benzoyl-phosphonsäureester der Formel (III) zwischen 0,2 und 0,5 Mol, vorzugsweise zwischen 0,25 und 0,4 Mol Hydridkomplex der Formel (IV) eingesetzt. In einer bevorzugten Ausführungsform von Verfahren 3) wird der Hydridkomplex der Formel (IV), vorzugsweise Natriumtetrahydridoborat, in einem Zweiphasengemisch aus Wasser und einem mit Wasser nicht mischbaren Lösungsmittel vorgelegt und der Benzoyl-phosphonsäureester, gegebenenfalls in einem mit Wasser nicht mischbaren Lösungsmittel gelöst, eindosiert. Das Reaktionsgemisch wird bis zum Reaktionsende gerührt, die organische Phase anschliessend abgetrennt, getrocknet, durch Vakuumdestillation vom Lösungsmittel befreit und «andestilliert», d.h. einige Zeit bei geringfügig erhöhter Temperatur evakuiert.

Die Produkte der Formel (II) werden so in öliger Form relativ rein erhalten.

Die bei Verfahren 3) als Ausgangsverbindungen zu verwendenden Benzoyl-phosphonsäureester sind durch Formel (III) definiert.

Vorzugsweise stehen darin $R^1$, $R^2$, $R^3$ und n für die bei den Verbindungen der Formel II als bevorzugt genannten Reste.

Als Beispiele für die Zwischenprodukte der Formel (III) seien genannt:

3-Brom-4-fluor-benzoyl-, 3-Phenoxy-benzoyl-, 4-Fluor-3-phenoxy-benzoyl-, 3-(4-Fluor-phenoxy)-benzoyl- und 4-Fluor-3-(4-fluor-phenoxy)-benzoyl-phosphonsäure-dimethylester und -diethylester sowie 2-Oxo-2-(3-phenoxy-benzoyl)-, 2-Oxo-2-(4-fluor-3-phenoxy-benzoyl)-, 2-Oxo-2-(3-(4-fluor-phenoxy)-benzoyl)- und 2-Oxo-2-(4-fluor-3-(4-fluor-phenoxy)-benzoyl)-5,5-dimethyl-1,3,2-dioxaphosphorinan.

Benzoylphosphonsäureester sind zum Teil Gegenstand einer nicht vorveröffentlichten Patentanmeldung (vgl. P 2 916 224/Le A 19 599). Man erhält sie durch Umsetzung von Phosphorigsäureester der Formel V

$$(R^3O)_2P{-}OR^4 \qquad (V)$$

in welcher

$R^3$ die oben angegebene Bedeutung hat und

$R^4$ für Methyl oder Ethyl steht,

mit Benzoesäurehalogeniden der Formel VI

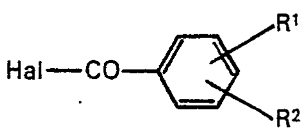

(VI)

in welcher

$R^1$ und $R^2$ die oben angegebenen Bedeutungen haben und

Hal für Fluor, Chlor oder Brom, vorzugsweise für Chlor steht,

bei Temperaturen zwischen 0 und 150°C, vorzugsweise zwischen 20 und 100°C.

In den Formeln (V) und (VI) haben $R^1$, $R^2$ und $R^3$ die gleichen bevorzugten Bedeutungen wie in Formel (II).

Als Beispiele für die Phosphorigsäureester der Formel (V) seien Trimethylphosphit, Triethylphosphit und 2-Ethoxy-5,5-dimethyl-1,3,2-dioxaphosphorinan genannt.

Als Beispiele für die Benzoesäurehalogenide der Formel (IV) seien genannt:

3-Brom-4-fluor-benzoylchlorid, 3-Phenoxy-benzoylchlorid, 4-Fluor-3-phenoxy-benzoylchlorid, 3-(4-Fluor-phenoxy)-benzoylchlorid und 4-Fluor-3-(4-fluor-phenoxy)-benzoylchlorid. Diese Verbindungen sind zum Teil bekannt oder lassen sich analog zu bekannten Verfahren herstellen.

So ist 3-Phenoxybenzoylchlorid bekannt aus GB-PS 1 052 390.

Die noch nicht in der Literatur beschriebenen fluorsubstituierten Phenoxybenzoesäurehalogenide erhält man, wenn man gemäss nachstehendem Reaktionsschema entsprechend substituierte Brombenzoesäuren (bzw. deren Alkalisalze) mit Alkaliphenolaten in Gegenwart eines Katalysators, wie z.B. Kupferoxid, und unter Verwendung von Phenol als Verdünnungsmittel bei Temperaturen zwischen 100 und 250°C, vorzugsweise zwischen 150 und 200°C umsetzt (vergleiche die nicht vorveröffentlichte Patentanmeldung P 2 915 738/Le A 19 590) und die hierbei gebildeten Phenoxybenzosäuren mit einem Halogenierungsmittel, wie z.B. Thionylchlorid, bei Temperaturen zwischen 20 und 100°C umsetzt:

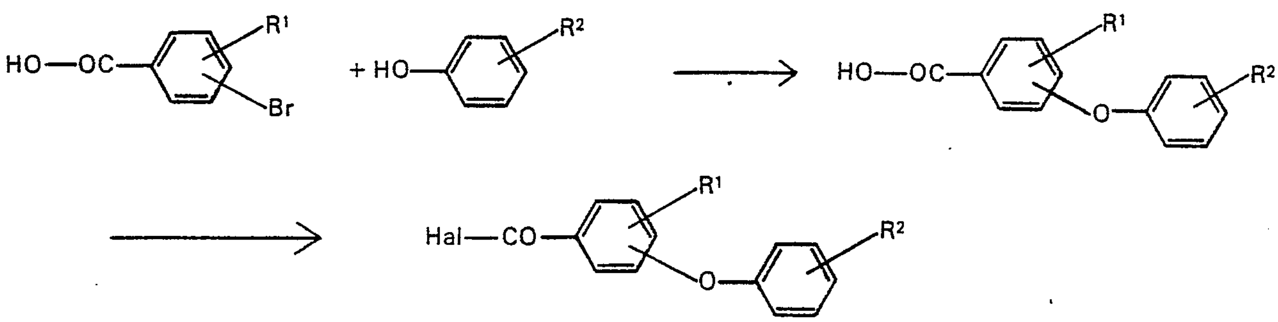

Die nach dem erfindungsgemässen Verfahren herzustellenden Benzaldehyde können als Zwischenprodukte zur Herstellung von Schädlingsbekämpfungsmitteln verwendet werden (Vgl. DE-OS 2 621 433).

Beispiel 1:

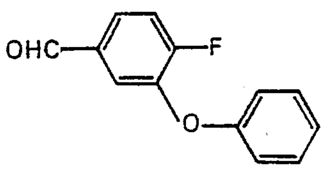

98 g (0,3 Mol) α-Hydroxy-4-fluor-3-phenoxy-benzyl-phosphonsäure-dimethylester und 200 ml Toluol werden zu einer unter einer Stickstoffatmosphäre befindlichen Lösung von 12 g (0,3 Mol) Natriumhydroxid in 200 ml Wasser gegeben. Das Reaktionsgemisch wird etwa 3 Stunden gerührt, wobei der pH-Wert auf etwa 9 zurückgeht. Die Toluolphase wird abgetrennt, getrocknet, filtriert und eingeengt. Das zurückbleibende Produkt wird durch Vakuumdestillation gereinigt. Man erhält 48 g (74% der Theorie) 4-Fluor-3-phenoxy-benzaldehyd vom Siedepunkt 86°C/0,01 mbar.

Elementaranalyse:

Ber.: C 72,2% H 4,2%
Gef.: C 71,3% H 4,2%

Beispiel 2:

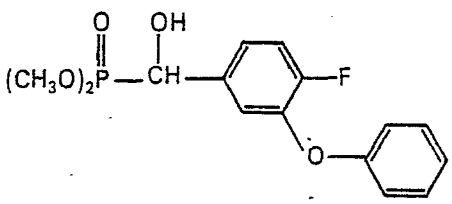

Eine Lösung von 130 g (0,4 Mol) 4-Fluor-3-phenoxy-benzoyl-phosphonsäure-dimethylester in 200 ml Methylenchlorid wird zu einer auf 0°C abgekühlten, intensiv gerührten Mischung aus 4,8 g (0,12 Mol) Natriumtetrahydridoborat, 200 ml Methylenchlorid und 400 ml Wasser tropfenweise gegeben. Das Reaktionsgemisch wird 90 Minuten bei 0°C gerührt, dann trennt man die organische Phase ab und extrahiert die wässrige Phase mit 200 ml Methylenchlorid. Die vereinigten Methylenchlorid-Phasen werden getrocknet, filtriert und das Lösungsmittel wird sorgfältig abdestilliert. Man erhält 120 g (92% der Theorie) α-Hydroxy-4-fluor-3-phenoxy-benzyl-phosphonsäure-dimethylester als zähflüssiges Öl.

Elementaranalyse:

Ber.: C 55,2% H 4,9% P 9,5%
Gef.: C 55,0% H 4,9% P 9,4%

Beispiel 3:

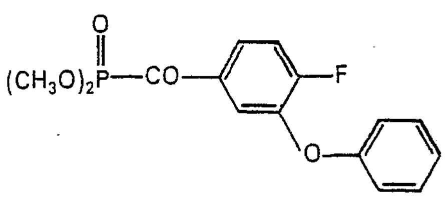

62 g Trimethylphosphit werden bei einer Innentemperatur von 60 bis 65°C zu 126 g (0,5 Mol) 4-Fluor-3-phenoxybenzoylchlorid tropfenweise gegeben. Das Reaktionsgemisch wird eine Stunde bei 80°C gerührt und evakuiert. Man erhält 158 g (97% der Theorie) 4-Fluor-3-phenoxybenzoyl-phosphonsäure-dimethylester als zähflüssiges Öl.

Elementaranalyse:

Ber.: C 55,6% H 4,3% P 9,6%
Gef.: C 55,0% H 4,3% P 9,7%

Beispiel 4:

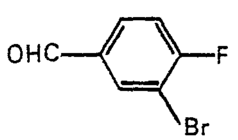

Ein Gemisch aus 31 g (0,1 Mol) α-Hydroxy-3-brom-4-fluor-benzyl-phosphonsäure-dimethylester, 4 g (0,1 Mol) Natriumhydroxid, 80 ml Wasser und 80 ml Ligroin wird zwei Stunden bei Raumtemperatur gerührt. Dann wird die organische Phase abgetrennt, getrocknet und filtriert. Vom Filtrat wird das Lösungsmittel unter vermindertem Druck sorgfältig abdestilliert, woraufhin das Produkt allmählich kristallisiert. Man erhält 18 g (89% der Theorie) 3-Brom-4-fluor-benzaldehyd vom Schmelzpunkt 32–33°C.

Beispiel 5:

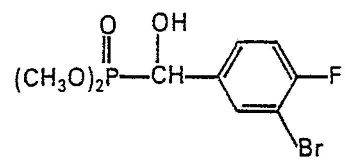

Eine Lösung von 94 g (0,3 Mol) 3-Brom-4-fluorbenzoyl-phosphonsäure-dimethylester in 100 ml Methylenchlorid wird zu einer auf 0°C gekühlten, intensiv gerührten Mischung aus 5 g Natriumtetrahydridoborat, 150 ml Methylenchlorid und 300 ml Wasser tropfenweise gegeben. Das Reaktionsgemisch wird 90 Minuten bei 0°C gerührt, dann trennt man die organische Phase ab und extrahiert die wässrige Phase mit 200 ml Methylenchlorid. Die vereinigten Methylenchlorid-Phasen werden getrocknet und filtriert. Vom Filtrat wird das Lösungsmittel unter vermindertem Druck sorgfältig abdestilliert. Nach Anreiben mit Ligroin kristallisiert das Produkt. Man erhält 75 g (80% der Theorie) α-Hydroxy-3-brom-4-fluor-benzyl-phosphonsäure-dimethylester vom Schmelzpunkt 91–93°C.

Beispiel 6:

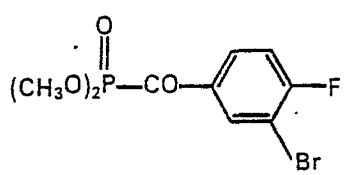

38 g Trimethylphosphit werden bei einer Innentemperatur zwischen 35 und 40°C zu einer Lösung von 72 g (0,3 Mol) 3-Brom-4-fluor-benzoylchlorid in 100 ml Methylenchlorid gegeben und das Reaktionsgemisch wird drei Stunden gerührt. Dann wird das Lösungsmittel unter vermindertem Druck sorgfältig abdestilliert. Man erhält 92 g (98% der Theorie) 3-Brom-4-fluor-benzoyl-phosphonsäure-dimethylester vom Siedepunkt 122°C/0,01 mbar.

**Patentanspruch**

1. Verfahren zur Herstellung von substituierten Benzaldehyden der Formel I

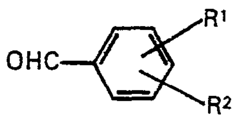

(I)

in welcher
$R^1$ für Wasserstoff oder Halogen steht und
$R^2$ für Halogen, oder gegebenenfalls halogensubstituiertes Phenoxy steht,
indem man α-Hydroxy-benzyl-phosphonsäureester der Formel II

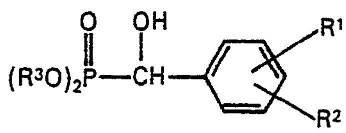

(II)

in welcher
$R^1$ und $R^2$ die oben angegebenen Bedeutungen haben und die beiden Reste
$R^3$ einzeln für Alkyl oder Phenyl oder zusammen für Alkandiyl (Alkylen) stehen,
mit Alkali- oder Erdalkalihydroxiden in einem Zweiphasensystem aus Wasser und einem mit Wasser nicht mischbaren Lösungsmittel bei Temperaturen zwischen 0 und 100°C umsetzt.

**Claim**

Process for the preparation of substituted benzaldehydes of the formula I

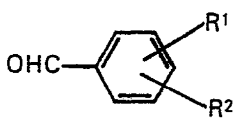

(I)

in which
$R^1$ represents hydrogen or halogen and
$R^2$ represents halogen or optionally halogen-substituted phenoxy,
by reacting α-hydroxy-benzyl-phosphonic acid esters of the formula II

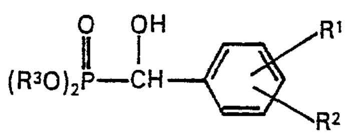

(II)

in which
$R^1$ and $R^2$ have the meanings indicated above and the two radicals
$R^3$ individually represent alkyl or phenyl or together represent alkanediyl (alkylene),
with alkali metal hydroxides or alkaline earth metal hydroxides in a two-phase system of water and a water-immiscible solvent at temperatures between 0 and 100°C.

**Revendication**

Procédé de préparation de benzaldéhydes substitués de formule I

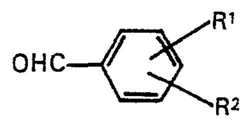

(I)

dans laquelle:
$R^1$ représente l'hydrogène ou un halogène et
$R^2$ représente un halogène ou un groupe phénoxy éventuellement substitué par des halogènes,
par réaction d'esters α-hydroxy-benzyl-phosphoniques de formule II

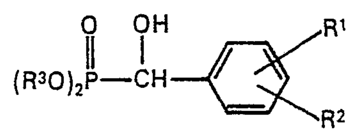

(II)

dans laquelle $R^1$ et $R^2$ ont les significations indiquées ci-dessus et les deux symboles $R^3$ représentent, individuellement, des groupes alkyle ou phényle ou, ensemble, un groupe alcane-diyle (alkylène); avec des hydroxydes alcalins ou alcalino-terreux dans un système à deux phases consistant en eau et un solvant non miscible à l'eau à des températures de 0 à 100°C.